(19) Europäisches Patentamt
European Patent Office
Office européen des brevets

(11) EP 3 107 894 B1

(12) EUROPÄISCHE PATENTSCHRIFT

(45) Veröffentlichungstag und Bekanntmachung des Hinweises auf die Patenterteilung:
**12.02.2020 Patentblatt 2020/07**

(21) Anmeldenummer: **15705911.4**

(22) Anmeldetag: **18.02.2015**

(51) Int Cl.:
***C07C 309/58*** *(2006.01)*

(86) Internationale Anmeldenummer:
**PCT/EP2015/000351**

(87) Internationale Veröffentlichungsnummer:
**WO 2015/124289 (27.08.2015 Gazette 2015/34)**

# (54) FLUORTENSIDE

FLUORINATED TENSIDES

TENSIOACTIFS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.02.2014 EP 14000617**

(43) Veröffentlichungstag der Anmeldung:
**28.12.2016 Patentblatt 2016/52**

(73) Patentinhaber: **Merck Patent GmbH**
**64293 Darmstadt (DE)**

(72) Erfinder:
- **FRIEDRICH, Reiner**
  **64342 Seeheim-Jugenheim (DE)**
- **SCHOOREN, Fanny**
  **64372 Ober-Ramstadt (DE)**

(56) Entgegenhaltungen:
**WO-A2-02/103103**
**US-A- 3 980 715**
**US-A- 4 049 668**
**US-A- 4 140 709**
**US-A1- 2007 051 916**
**US-A1- 2009 326 083**

- **ZHAO-TIE LIU ET AL: "Water in Carbon Dioxide Microemulsions with Fluorinated Analogues of AOT", LANGMUIR, Bd. 17, Nr. 2, 1. Januar 2001 (2001-01-01) , Seiten 274-277, XP55183701, ISSN: 0743-7463, DOI: 10.1021/la000947e**
- **A.R. PITT ET AL: "The relationship between surfactant structure and limiting values of surface tension, in aqueous gelatin solution, with particular regard to multilayer coating", COLLOIDS AND SURFACES A: PHYSICOCHEMICAL AND ENGINEERING ASPECTS, Bd. 114, 1. August 1996 (1996-08-01), Seiten 321-335, XP55183642, ISSN: 0927-7757, DOI: 10.1016/0927-7757(96)03593-5**

EP 3 107 894 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Gegenstand der vorliegenden Erfindung sind neue Verbindungen mit fluorierten Endgruppen, deren Verwendung als oberflächenaktive Substanzen und Mittel enthaltend diese Verbindungen.

Fluorhaltige Tenside besitzen aufgrund ihrer speziellen Oberflächenaktivität einzigartige Anwendungseigenschaften. Fluortenside, deren statische Oberflächenspannung sehr niedrig ist (16-20 mN/m), sind in verschiedensten Anwendungen einsetzbar und tragen z.B. zur verbesserten Benetzung von Oberflächen bei. So werden sie z.B. als Grenzflächenvermittler bzw. Emulgator oder Viskositätsminderer in Farben, Lacken oder Klebstoffen verwendet.

Klassische Fluortenside sind aus langkettigen, perfluorierten Alkylketten (C6-C8) aufgebaut und gelten potentiell als bioakkumulativ und toxisch. In der Regel enthalten Fluortenside jedoch Perfluoralkylsubstituenten, die in der Umwelt durch biologische und andere Oxidationsprozesse zu Perfluoralkancarbonsäuren und -sulfonsäuren abgebaut werden. Diese gelten als persistent und stehen z. T. im Verdacht gesundheitliche Schäden zu verursachen (G. L. Kennedy, Jr., J. L. Butenhoff, G. W. Olsen, J. C. O'Connor, A. M. Seacat, R. G. Perkins, L. B. Biegel, S. R. Murphy, D. G. Farrar, Critical Reviews in Toxicology 2004, 34, 351-384). Längerkettige Perfluoralkancarbonsäuren und -sulfonsäuren reichern sich zudem in der Nahrungskette an. Kürzerkettige Fluorbausteine sind von ihrem ökotoxikologischen Profil günstiger, zeigen jedoch in ihren Anwendungsbereichen oft schlechtere Eigenschaften.

In WO 03/010128 werden Perfluoralkyl-substituierte Amine, Säuren, Aminosäuren und Thioethersäuren beschrieben, die eine $C_{3-20}$-Perfluoralkyl-Gruppe aufweisen. Aus JP-A-2001/133984 sind oberflächenaktive Verbindungen mit Perfluoralkoxy-Ketten bekannt, die sich zum Einsatz in Antireflex-Beschichtungen eignen. Aus JP-A-09/111286 ist die Verwendung von Perfluorpolyethertensiden in Emulsionen bekannt. In WO 2006/072401 und WO 2010/003567 werden oberflächenaktive Verbindungen mit Trifluormethoxygruppen beschrieben. Verbindungen mit speziellen Fluoralkylgruppen sind in US 7,635,789, US 2008/0093582, JP 2004-18394 und WO 2010/002623 beschrieben. Teilfluorierte Verbindungen sind in US 7,737,307, EP 1 522 536 und WO 2010/002622 beschrieben.

Spezielle Anwendungen von Sulfosuccinaten und/oder Sulfotricarballylaten mit verschiedenen fluorierten Seitenketten sind in US 4,968,599 und US 4,988,610 sowie US 6, 890, 608 beschrieben und in A.R. Pitt et al., Colloids and Surfaces A: Physicochemical and Engineering Aspects, 1996, 114, 321-335; A.R. Pitt, Progr. Colloid Polym. Sci, 1997, 103, 307-317 und Z.-T. Liu et al., Ind. Eng. Chem. Res. 2007, 46, 22-28. Weitere Fluortenside, insbesondere Succinate und Tricarballylate mit fluorierten Alkylgruppen, sind in WO 2009/149807, WO 2010/003567, WO 2010/149262, WO 2011/082770 und WO 2012/084118 beschrieben.

Weiterhin besteht Bedarf nach alternativen oberflächenaktiven Substanzen, die vorzugsweise beim Abbau nicht zu langkettigen persistenten Verbindungen abbauen.

Es wurden nun neue Verbindungen gefunden, die als oberflächenaktive Substanzen geeignet sind und bevorzugt einen oder mehrere der o.g. Nachteile nicht aufweisen.

Ein erster Gegenstand der vorliegenden Erfindung sind Verbindungen der Formel (I) oder (I') gemäß Anspruch 1

$$Z_nC_cD_dSpacer_mX_x \qquad (I)$$

$$(Z_nC_cD_d)_nSpacer_mX_x \qquad (I')$$

wobei

- Z = $R_f$-O-CHF-$CF_2$-$CR^1R^2$-O- oder
  $R_f$-O-$(CF_2)_{1-4}$-CHF-$CF_2$-$CR^1R^2$-O- oder
  $R_f$-O-$(CF_2)_{1-4}$-O-CHF-$CF_2$-$CR^1R^2$-O- oder
  $R_f$-O-$(CF(CF_3)$-$CF_2)_{1-4}$-CHF-$CF_2$-$CR^1R^2$-O- oder
  $R_f$-O-$(CF(CF_3)$-$CF_2)_{1-4}$-O-CHF-$CF_2$-$CR^1R^2$-O- oder
  $R_f$-O-$(CF_2$-$O)_{1-4}$-CHF-$CF_2$-$CR^1R^2$-O- oder
  $R_f$-O-$(CF_2$-$CF_2$-$O)_{1-4}$-CHF-$CF_2$-$CR^1R^2$-O- oder
  $R_f$-O-$(CF_2$-$O)_{1-4}$-$(CF_2$-$CF_2$-$O)_{1-4}$-CHF-$CF_2$-$CR^1R^2$-O-
- n und n' = 1, 2, 3,4, 5 oder 6, bevorzugt 2-4, insbesondere 2-3,
- $R_f$ = fluoriertes Alkyl, linear oder verzweigt, bevorzugt fluoriertes C1-C6-Alkyl, besonders bevorzugt perfluoriertes C1-C4-Alkyl, , insbesondere perfluoriertes C1-C3-Alkyl,
- $R^1$ = H oder Alkyl, bevorzugt C1-C4, insbesondere -$CH_3$ oder -$CH_2$-$CH_3$
- $R^2$ = H oder Alkyl, bevorzugt C1-C4, insbesondere -$CH_3$,
- C = -$CR'_2$-$CR''_2$-O-,
- c = 0 oder eine ganze Zahl aus dem Bereich von 1 bis 100,
- R' und R" = unabhängig voneinander H oder Alkyl, insbesondere H oder -$CH_3$ oder -$CH_2$-$CH_3$
- D = -CO-

d = 0 oder 1,
Spacer = eine gesättigte oder ungesättigte, verzweigte oder unverzweigte, ggf. Heteroatome enthaltende, Kohlenwasserstoffeinheit,
m = 0 oder 1,
X eine anionische, kationische, nicht-ionische oder amphotere hydrophile Gruppe gemäß Anspruch 1 ist,
und x = 1, 2, 3 oder 4, bevorzugt 1 ist

Bevorzugt entsprechen erfindungsgemäße Verbindungen der Formel (I')

$$(ZC_cD_d)_nSpacer_mX_x \qquad (I)$$

wobei alle Variablen die genannten Bedeutungen.
Bei den Kohlenwasserstoff-Einheiten des Spacers der Verbindungen der Formeln (I) und (I') kann es sich um aliphatische oder aromatische, gegebenenfalls mit Heteroatomen versehene Einheiten handeln. Vorzugsweise ist die Gruppe Z in den oberflächenaktiven Verbindungen dabei an eine gesättigte, verzweigte oder unverzweigte Kohlenwasserstoff-Einheit, bevorzugt an eine gesättigte, verzweigte oder unverzweigte Alkylengruppe, wobei ein oder mehrere nicht benachbarte C-Atome durch O oder N, bevorzugt O, ersetzt sein können, gebunden. In einer Erfindungsvariante wird als bevorzugte Heteroatome enthaltende Kohlenwasserstoffeinheit eine Polyethylen- oder Polypropylenglykoleinheit verwendet.
In einer Erfindungsvariante kommt die Gruppe Z in der oberflächenaktiven Verbindung mehrfach vor, bevorzugt zweimal oder dreimal. In einer anderen Erfindungsvariante kommt die Gruppe Z in der oberflächenaktiven Verbindung nur einmal vor. In einer Erfindungsvariante können Verbindungen der Formeln (I) und (I') verschiedene Gruppe Z enthalten.
Bevorzugt sind Verbindungen der Formeln (I) und (I'), in denen n = 2-3 und x = 1 ist.
In einer Erfindungsvariante ist m bevorzugt 1.
In den Verbindungen der Formeln (I) und (I') ist Z gleich:

$R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder
$R_f$-O-$(CF(CF_3)$-$CF_2)_{1-4}$-CHF-$CF_2$-$CR^1R^2$-O- oder
$R_f$-O-$(CF(CF_3)$-$CF_2)_{1-4}$-O-CHF-$CF_2$-$CR^1R^2$-O- oder
$R_f$-O-$(CF_2$-$O)_{1-4}$-CHF-$CF_2$-$CR^1R^2$-O- oder
$R_f$-O-$(CF_2$-$CF_2$-$O)_{1-4}$-CHF-$CF_2$-$CR^1R^2$-O- oder
$R_f$-O-$(CF_2$-$O)_{1-4}$-$(CF_2$-$CF_2$-$O)_{1-4}$-CHF-$CF_2$-$CR^1R^2$-O- ist.

Besonders bevorzugt sind Verbindungen der Formeln (I) und (I') in denen Z gleich:

$R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder
$R_f$-O-$(CF(CF_3)$-$CF_2)_{1-4}$-CHF-$CF_2$-$CR^1R^2$-O- oder
$R_f$-O-$(CF(CF_3)$-$CF_2)_{1-4}$-O-CHF-$CF_2$-$CR^1R^2$-O- oder
$R_f$-O-$(CF_2$-$O)_{1-4}$-CHF-$CF_2$-$CR^1R^2$-O- oder
$R_f$-O-$(CF_2$-$CF_2$-$O)_{1-4}$-CHF-$CF_2$-$CR^1R^2$-O- oder
$R_f$-O-$(CF_2$-$O)_{1-4}$-$(CF_2$-$CF_2$-$O)_{1-4}$-CHF-$CF_2$-$CR^1R^2$-O- ist,
insbesondere = $R_f$-O-CHF-$CF_2$-$CR^1R^2$-O- oder
$R_f$-O-$(CF_2)_{1-4}$-CHF-$CF_2$-$CR^1R^2$-O-, mit $R^1$ und $R^2$ unabhängig voneinander = H, -$CH_3$ oder -$CH_2$-$CH_3$. Bevorzugt sind hierbei Verbindungen, in denen $R^1$ und $R^2$ = H und solche Verbindungen, in denen $R^1$ oder $R^2$ = H.

Bevorzugt sind Verbindungen der Formeln (I) und (I'), in denen Rfgleich: $CF_3$- oder $CF_3$-$CF_2$- oder $CF_3$-$CF_2$-$CF_2$-ist.
Im Besonderen sind Verbindungen der Formeln (I) und (I') bevorzugt, in denen n = 2-3, x = 1, Rf gleich $CF_3$-, $CF_3$-$CF_2$- oder $CF_3$-$CF_2$-$CF_2$- ist und Z gleich:

$R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder

$R_f$-O-(CF($CF_3$)-$CF_2$)$_{1-4}$-CHF-$CF_2$-$CR^1R^2$-O- oder
$R_f$-O-(CF($CF_3$)-$CF_2$)$_{1-4}$-O-CHF-$CF_2$-$CR^1R^2$-O- oder
$R_f$-O-($CF_2$-O)$_{1-4}$-CHF-$CF_2$-$CR^1R^2$-O- oder
$R_f$-O-($CF_2$-$CF_2$-O)$_{1-4}$-CHF-$CF_2$-$CR^1R^2$-O- oder
$R_f$-O-($CF_2$-O)$_{1-4}$-($CF_2$-$CF_2$-O)$_{1-4}$-CHF-$CF_2$-$CR^1R^2$-O- ist.

Hierbei ist Z insbesondere = $R_f$-O-CHF-$CF_2$-$CR^1R^2$-O- oder $R_f$-O-($CF_2$)$_{1-4}$-CHF-$CF_2$-$CR^1R^2$-O-, mit $R^1$ und $R^2$ unabhängig voneinander = H, -$CH_3$ oder -$CH_2$-$CH_3$. Bevorzugt sind hierbei Verbindungen, in denen $R^1$ und $R^2$ = H und solche Verbindungen, in denen $R^1$ oder $R^2$ = H.

Hierbei sind Verbindungen bevorzugt, in denen der Spacer eine gesättigte, verzweigte oder unverzweigte Kohlenwasserstoff-Einheit ist und m = 1.

Bevorzugt ist C = -$CH_2$-CHR"-O- oder -CHR'-$CH_2$-O-,, mit c = 0 oder eine ganze Zahl aus dem Bereich von 1 bis 100, bevorzugt = 1-100, und R' und R" = H oder Alkyl, insbesondere H oder -$CH_3$ oder -$CH_2$-$CH_3$.

Besonders bevorzugt sind Verbindungen in denen die genannten Variablen die bevorzugten Bedeutungen haben, insbesondere solche, in denen alle Variablen die bevorzugten Bedeutungen haben.

In den erfindungsgemäßen Verbindungen ist X eine anionische, kationische, nicht-ionische oder amphotere hydrophile Gruppe ausgewählt aus den Gruppen gemäß Anspruch 1.

Eine anionische Gruppe X ist ausgewählt aus -$COO^-$, -$SO_3^-$, -$OSO_3^-$, -$PO_3^{2-}$, -$OPO_3^{2-}$, -($OCH_2CH_2$)$_s$-O-($CH_2$)$_t$-$COO^-$, -($OCH_2CH_2$)$_s$-O-($CH_2$)$_t$-$SO_3^-$,-($OCH_2CH_2$)$_s$-O-($CH_2$)$_t$-$OSO_3^-$, -($OCH_2CH_2$)$_s$-O-($CH_2$)$_t$-$PO_3^{2-}$, -($OCH_2CH_2$)$_s$-O-($CH_2$)$_t$-$OPO_3^{2-}$ oder aus den Formeln A bis C,

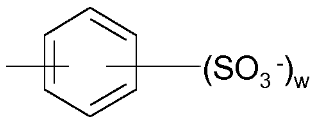
A

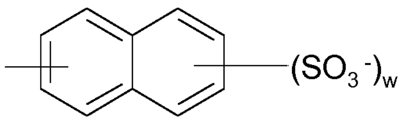
B

oder

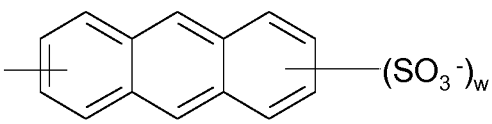
C

wobei s steht für eine ganze Zahl aus dem Bereich von 1 bis 1000, t steht für eine ganze Zahl ausgewählt aus 1, 2, 3 oder 4 und w steht für eine ganze Zahl ausgewählt aus 1, 2 oder 3.

Zu den bevorzugten anionischen Gruppen gehören dabei insbesondere -$COO^-$, -$SO_3^-$, -$OSO_3^-$, -$PO_3^{2-}$, -$OPO_3^{2-}$, die Teilformel A, sowie -($OCH_2CH_2$)$_s$-O-($CH_2$)$_t$-$COO^-$, -($OCH_2CH_2$)$_s$-O-($CH_2$)$_t$-$SO_3^-$ und -($OCH_2CH_2$)$_s$-O-($CH_2$)$_t$-$OSO_3^-$, wobei jede einzelne dieser Gruppen für sich genommen bevorzugt sein kann.

Zu den ganz besonders bevorzugten anionischen Gruppen gehören dabei -$SO_3^-$, -$OSO_3^-$, -$PO_3^{2-}$, oder $OPO_3^{2-}$. Insbesondere eine Sulfonatgruppe -$SO_3^-$ ist bevorzugt.

Bevorzugtes Gegenion für anionische Gruppen X ist ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt sind $Na^+$, $K^+$, $Li^+$ und $NH_4^+$, besonders bevorzugt $Na^+$.

Eine kationische Gruppe X ist ausgewählt aus -$NR^1R^2R^{3+}$ $Z^-$, -$PR^1R^2R^{3+}$ $Z^-$,

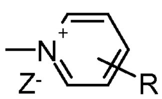
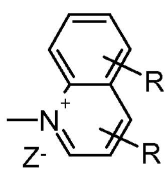
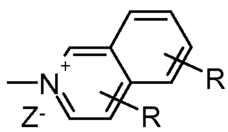

wobei R steht für H oder $C_{1-4}$-Alkyl in beliebiger Position,

$Z^-$ steht für $Cl^-$, $Br^-$, $I^-$, $CH_3SO_3^-$, $CF_3SO_3^-$, $CH_3PhSO_3^-$, $PhSO_3^-$
$R^1$, $R^2$ und $R^3$ jeweils unabhängig voneinander stehen für H, $C_{1-30}$-Alkyl, Ar oder $-CH_2Ar$ und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

Zu den bevorzugten kationischen Gruppen gehören dabei insbesondere $-NR^1R^2R^{3+}$ $Z^-$ und

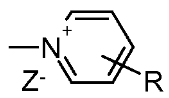

wobei jede einzelne dieser Gruppen für sich genommen bevorzugt sein kann.

Eine nicht-ionische Gruppe ist ausgewählt aus: lineares oder verzweigtes Alkyl, wobei ein oder mehrere nicht benachbarte C-Atome durch O, S, und/oder N ersetzt sind, -OH, -SH, $-O-(Glycosid)_{o'}$, $-S-(Glycosid)_{o'}$, $-OCH_2-CHOH-CH_2-OH$, $-O\ CH_2Ar(-NCO)_{p'}$, $-OAr(-NCO)_{p'}$, Aminoxid,

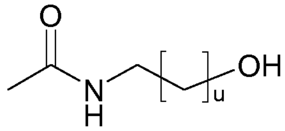
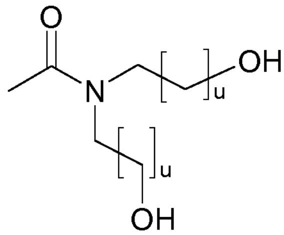
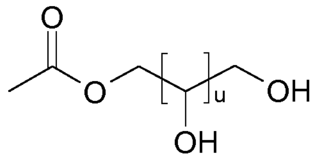

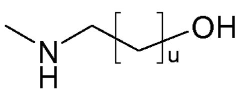
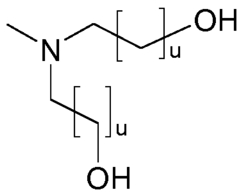
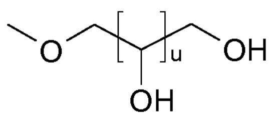

u steht für eine ganze Zahl aus dem Bereich von 1 bis 6, bevorzugt 1 bis 4
o' steht für eine ganze Zahl aus dem Bereich von 1 bis 10,
p' steht für 1 oder 2,
Ar steht für einen unsubstituierten, ein- oder mehrfach substituierten aromatischen Ring oder kondensierte Ringsysteme mit 6 bis 18 C-Atomen, worin auch ein oder zwei CH-Gruppen durch C=O ersetzt sein können und, Glycosid steht für ein verethertes Kohlenhydrat, vorzugsweise für ein mono- di-, tri- oder oligo-Glucosid.

Zu den bevorzugten nicht-ionischen Gruppen gehören dabei insbesondere lineares oder verzweigtes Alkyl, wobei ein oder mehrere nicht benachbarte C-Atome durch O, S und/oder N ersetzt sind, -OH und $-O-(Glycosid)_{o'}$.

Wenn X = Alkyl, wobei ein oder mehrere nicht benachbarte C-Atome durch O, S, und/oder N ersetzt sind, dann ist es bevorzugt gleich $R^4-(B-A)_{m''}-$ mit $R^4$ = H oder C1-4-Alkyl, insbesondere H oder $CH_3$, A = lineares oder verzweigtes Alkylen, bevorzugt mit 1 bis 10 Kohlenstoffatomen, insbesondere mit 1 bis 4 Kohlenstoffatomen, B = O oder S, bevorzugt O, und m'' = eine ganze Zahl bevorzugt aus dem Bereich von 1 bis 100, besonders bevorzugt 1 bis 30.

Insbesondere bevorzugt als nicht-ionische Gruppe X ist die Gruppe $R^4-(O-CH_2CHR^5)_{m''}-$ mit m'' = eine ganze Zahl aus dem Bereich von 1 bis 100, bevorzugt 1 bis 30, insbesondere 1-15, und $R^4$ und $R^5$ = H oder C1-4-Alkyl, insbesondere H oder $CH_3$. Insbesondere bevorzugt ist $R^4-(B-A)_{m''}$-eine Polyethylen- oder Polypropylenglykoleinheit.

Insbesondere bevorzugt als nicht-ionische Gruppe X ist die Gruppe $-CH(OH)-CH_2-NH-Sach$ mit Sach = verschiedene Zucker und die Gruppe $-Y-(CH_2-CH_2-O)_v-R^4$ mit Y = S, O oder NH, bevorzugt O, $R^4$ = H oder Alkyl, bevorzugt H oder $CH_3$, und v = 1-100, bevorzugt 1-20, insbesondere 1-15.

Eine amphotere Gruppe ist ausgewählt aus den funktionellen Gruppen der Acetyldiamine, der N-Alkylaminosäuren, der N-Alkylaminosulfonsäuren, der Betaine, der Sulfobetaine, insbesondere ausgewählt aus, wobei M steht für H oder ein Alkalimetall-Ion, vorzugsweise $Li^+$, $Na^+$ oder $K^+$:

| |
|---|
| 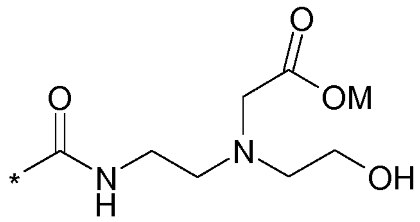 |
| 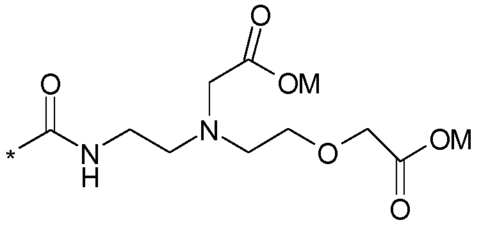 |
| 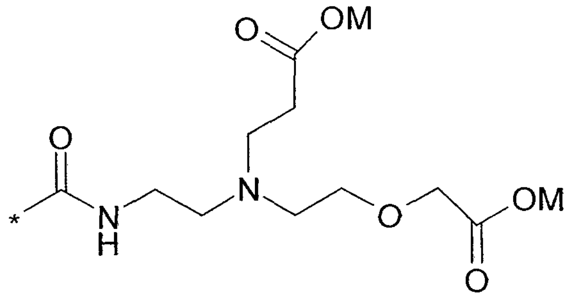 |
| $-NH-CH_2-COOM$; $-NH-CH_2-CH_2-COOM$ |
| 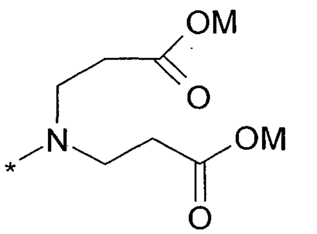 |
| $-[(C(=O)-NH-(CH_2)_{(1-8)}]_{(0\ oder\ 1)}-N^+R^1R^2-CH_2-COO^-$, wobei $R^1$ und $R^2$ jeweils unabhängig voneinander stehen für einen C1-8-Alkylrest, vorzugsweise Methyl oder Ethyl |
| $-C(=O)-NH-(CH_2)_{1-3}-N^+R^1R^2-CH_2-CH(OH)-CH_2-(O)_{(0\ oder\ 1)}-(S\ oder\ P)O_3^-$, wobei $R^1$ und $R^2$ jeweils unabhängig voneinander stehen für einen C1-8-Alkylrest, vorzugsweise Methyl oder Ethyl |

Besonders bevorzugte erfindungsgemäße Verbindungen sind solche, die als hydrophile Gruppe X eine der bevorzugten anionische Gruppen, der bevorzugten nicht-ionischen Gruppen oder der bevorzugten zwitterionischen Gruppen enthalten.

Insbesondere bevorzugt sind Verbindungen, die die Gruppen $-SO_3^-$, $-OSO_3^-$, $-PO_3^{2-}$ oder $OPO_3^{2-}$, Polyethylen- oder Polypropylenglykole, $-CH(OH)-CH_2-NH-Sach$, $-Y-(CH_2-CH_2-O)_v-R^4$, Betaine, oder Sulfobetaine enthalten. Bevorzugte Gegenionen sind hierbei $Na^+$, $K^+$ und $NH_4^+$, insbesondere $Na^+$. Insbesondere bevorzugt sind: $-SO_3^-$, Polyethylen- oder Polypropylenglykole, Sulfobetaine, die Gruppe $-CH(OH)-CH_2-NH-Sach$ und die Gruppe $-Y-(CH_2-CH_2-O)_v-R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-20, insbesondere 1-15. Besonders vorteilhaft sind Verbindungen mit X = $-SO_3^-$.

Besonders vorteilhaft sind Verbindungen der Formeln (I) und (I'), in denen eine oder mehrere der Variablen die bevorzugten Bedeutungen haben. Besonders vorteilhaft sind Verbindungen der Formeln (I) und (I'), in denen alle genannten Variablen die bevorzugten Bedeutungen haben.

Insbesondere bevorzugt sind Verbindungen der Formeln (II) bis (XI),

mit Y = $-(OCH_2-CHR'_2)_{0/1}-Z$

X = $Na^+$, $K^+$, $Li^+$, $NH^{4+}$, $N(CH_3)^{4+}$,
Q = Cl, Br, I
R' und R" = unabhängig voneinander H oder C1-C4 Alkyl,
bevorzugt ist Y = $-(OCH_2\text{-}CHR'_2)_{0/1}\text{-}Z$, mit R' = H oder C1-C2 Alkyl,

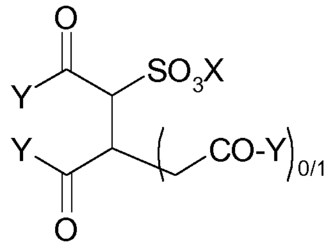

Formel (II)

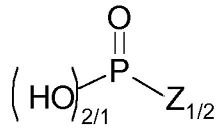

Formel (III)

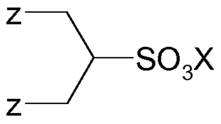

Formel (IV)

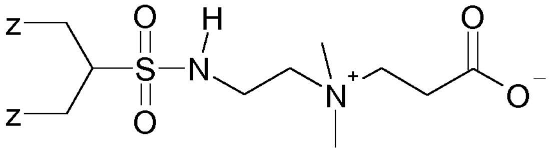

Formel (V)

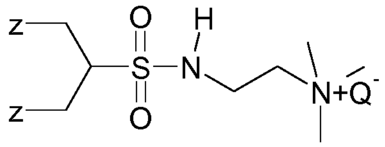

Formel (VI)

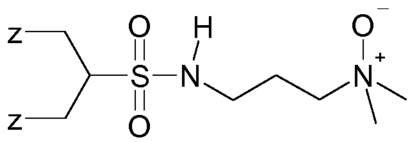

Formel (VII)

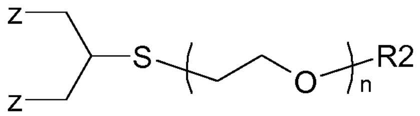

Formel (VIII)

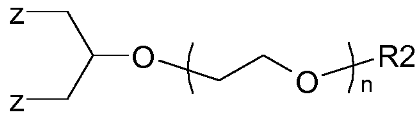

Formel (IX)

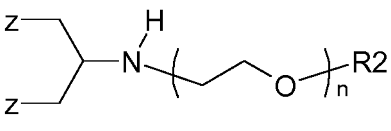

Formel (X)

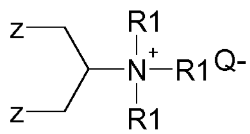

Formel (XI)

Insbesondere bevorzugt sind Verbindungen der Formeln (II) bis (XI) in denen eine oder mehrere der Variablen die bevorzugten Bedeutungen haben. Besonders die Varianten der Formeln (II) bis (XI) sind bevorzugt, in denen alle Variablen die bevorzugten Bedeutungen haben, vor Allem die besonders bevorzugten Bedeutungen. Besonders vorteilhaft sind Verbindungen der Formel (II), in denen alle genannten Variablen die bevorzugten Bedeutungen haben. Die in den Formeln (II) bis (XI) angegebenen bevorzugten hydrophilen Gruppen können auch durch andere hydrophile Gruppen ersetzt sein.

Besonders bevorzugte Verbindungen der Formeln (II) bis (XI) sind Verbindungen, in denen Rf gleich $CF_3$-,

$CF_3$-$CF_2$- oder $CF_3$-$CF_2$-$CF_2$- ist und Z gleich:

$R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder
$R_f$-O-$(CF(CF_3)$-$CF_2)_{1-4}$-CHF-$CF_2$-$CR^1R^2$-O- oder
$R_f$-O-$(CF(CF_3)$-$CF_2)_{1-4}$-O-CHF-$CF_2$-$CR^1R^2$-O- oder
$R_f$-O-$(CF_2$-$O)_{1-4}$-CHF-$CF_2$-$CR^1R^2$-O- oder
$R_f$-O-$(CF_2$-$CF_2$-$O)_{1-4}$-CHF-$CF_2$-$CR^1R^2$-O- oder
$R_f$-O-$(CF_2$-$O)_{1-4}$-$(CF_2$-$CF_2$-$O)_{1-4}$-CHF-$CF_2$-$CR^1R^2$-O- ist.

Hierbei ist Z insbesondere = $R_f$-O-CHF-$CF_2$-$CR^1R^2$-O- oder $R_f$-O-$(CF_2)_{1-4}$-CHF-$CF_2$-$CR^1R^2$-O-, mit $R^1$ und $R^2$ unabhängig voneinander = H, -$CH_3$ oder -$CH_2$-$CH_3$. Bevorzugt sind hierbei Verbindungen, in denen $R^1$ und $R^2$ = H und solche Verbindungen, in denen $R^1$ oder $R^2$ = H.

Hierbei sind Verbindungen bevorzugt, die als X die Gruppen -$SO_3^-$, -$OSO_3^-$, -$PO_3^{2-}$ oder $OPO_3^{2-}$, Polyethylen- oder Polypropylenglykole, -CH(OH)-$CH_2$-NH-Sach, -Y-$(CH_2$-$CH_2$-$O)_v$-$R^4$, Betaine, oder Sulfobetaine enthalten. Bevorzugte Gegenionen sind hierbei $Na^+$, $K^+$ und $NH_4^+$, insbesondere $Na^+$. Insbesondere bevorzugt sind: -$SO_3^-$, Polyethylen- oder Polypropylenglykole, Sulfobetaine, die Gruppe -CH(OH)-$CH_2$-NH-Sach und die Gruppe -Y-$(CH_2$-$CH_2$-$O)_v$-$R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-20, insbesondere 1-15. Besonders vorteilhaft sind Verbindungen mit X = -$SO_3^-$.

Bevorzugt sind vor allem Verbindungen der Formel (II) und auch Verbindungen der Formeln (II-a) (II-b), (II-c) und (II-d) in denen die Variablen die für die Formeln (I), (I') bzw. (II) angegebenen Bedeutungen haben, insbesondere die bevorzugten Bedeutungen.

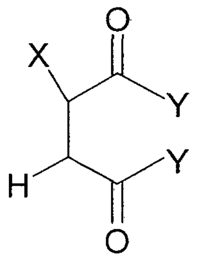
(II-a)

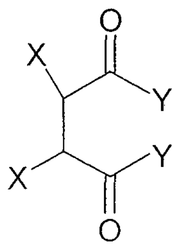
(II-b)

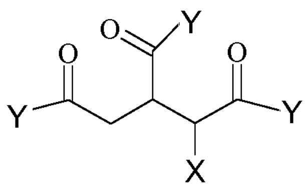
(II-c)

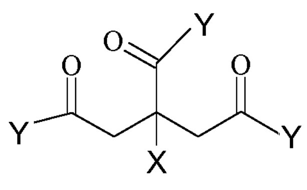
(II-d)

Beispiele besonders vorteilhafter Verbindungen sind Succinate und Tricarballylate, in denen Y, Z und X die

vorstehend beschriebene Bedeutung, insbesondere die bevorzugten Bedeutungen, haben.

Besonders bevorzugte Verbindungen der Formeln (II) und (IIa) bis (IId) sind Verbindungen, in denen Rf gleich $CF_3$-, $CF_3$-$CF_2$- oder $CF_3$-$CF_2$-$CF_2$- ist und Z gleich:

$R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder
$R_f$-O-$(CF(CF_3)$-$CF_2)_{1-4}$-CHF-$CF_2$-$CR^1R^2$-O- oder
$R_f$-O-$(CF(CF_3)$-$CF_2)_{1-4}$-O-CHF-$CF_2$-$CR^1R^2$-O- oder
$R_f$-O-$(CF_2$-$O)_{1-4}$-CHF-$CF_2$-$CR^1R^2$-O- oder
$R_f$-O-$(CF_2$-$CF_2$-$O)_{1-4}$-CHF-$CF_2$-$CR^1R^2$-O- oder
$R_f$-O-$(CF_2$-$O)_{1-4}$-$(CF_2$-$CF_2$-$O)_{1-4}$-CHF-$CF_2$-$CR^1R^2$-O- ist.

Hierbei ist Z insbesondere = $R_f$-O-CHF-$CF_2$-$CR^1R^2$-O- oder $R_f$-O-$(CF_2)_{1-4}$-CHF-$CF_2$-$CR^1R^2$-O-, mit $R^1$ und $R^2$ unabhängig voneinander = H, -$CH_3$ oder -$CH_2$-$CH_3$. Bevorzugt sind hierbei Verbindungen, in denen $R^1$ und $R^2$ = H und solche Verbindungen, in denen $R^1$ oder $R^2$ = H.

Hierbei sind Verbindungen bevorzugt, die als X die Gruppen -$SO_3^-$, -$OSO_3^-$, -$PO_3^{2-}$ oder $OPO_3^{2-}$, Polyethylen- oder Polypropylenglykole, -CH(OH)-$CH_2$-NH-Sach, -Y-$(CH_2$-$CH_2$-$O)_v$-$R^4$, Betaine, oder Sulfobetaine enthalten. Bevorzugte Gegenionen sind hierbei $Na^+$, $K^+$ und $NH_4^+$, insbesondere $Na^+$. Insbesondere bevorzugt sind: -$SO_3^-$, Polyethylen- oder Polypropylenglykole, Sulfobetaine, die Gruppe -CH(OH)-$CH_2$-NH-Sach und die Gruppe -Y-$(CH_2$-$CH_2$-$O)_v$-$R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-20, insbesondere 1-15. Besonders vorteilhaft sind Verbindungen mit X = -$SO_3^-$.

Die erfindungsgemäßen Verbindungen sind nach dem Fachmann bekannten Verfahren herstellbar.

Die erfindungsgemäßen Verbindungen der Formel (II) können bevorzugt durch Veresterung von Maleinsäure und Aconitsäure bzw. deren Anhydriden oder Säurechloriden mit einem oder mehreren Alkoholen der Formel (XII), wobei Y die vorstehend beschriebenen Bedeutungen insbesondere die bevorzugten Bedeutungen hat

Y-H (XII)

und anschließender Addition an die Doppelbindung zur Einführung der Gruppierung X hergestellt werden.

Ein weiterer Gegenstand der Erfindung sind somit die entsprechenden Maleinsäure- und Aconitsäureester der Formeln (XIII) bzw. (XIV):

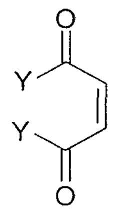

(XIII)

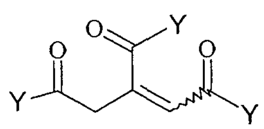

(XIV)

Die Variablen haben in den Formeln (XIII) und (XIV) die vorstehend beschriebenen Bedeutungen, insbesondere auch die bevorzugten Bedeutungen. Die Formel (XIV) gibt das Vorliegen von Z/E-Doppelbindungs-Isomeren wieder.

Die erfindungsgemäßen Verbindungen können auch bevorzugt durch Veresterung von Hydroxybernsteinsäure und Zitronensäure mit einem oder mehreren Alkoholen der Formel (XII) und anschließende Funktionalisierung der Hydroxygruppen zur Einführung der Gruppierung X hergestellt werden.

Die verwendeten Alkohole sind kommerziell erhältlich und/oder ihre Herstellung ausgehend von kommerziell erhältlichen Edukten ist dem Fachmann geläufig oder sie können in Analogie zu bekannten Syntheseverfahren hergestellt werden, z. B. radikalisch Addition siehe: A. A. Il'in et al., Russian Journal of Applied Chemistry, 2007, Vol. 80, No. 3,

pp. 405-418.

Die Synthese erfindungsgemäßer Succinate bzw. Tricarballylate erfolgt bevorzugt wie in WO 2014/012661 beschrieben in einer zweistufigen Synthese über die entsprechenden Maleate oder Hydroxysuccinate bzw. die entsprechenden Aconit- oder Zitronensäureester. Die Herstellung weiterer erfindungsgemäßer Verbindungen der Formel (II) kann analog zu den oben gezeigten beispielhaften Reaktionen erfolgen. Die Herstellung weiterer erfindungsgemäßer Verbindungen der Formel (II) kann auch nach anderen dem Fachmann an sich aus der Literatur bekannten Methoden erfolgen. Insbesondere andere Veresterungkatalysatoren können verwendet werden. Diese genannten Synthesen sind in WO 2010/149262, WO 2011/082770 und WO 2012/084118 beschrieben. Die Offenbarungen in den zitierten Literaturstellen gehören hiermit ausdrücklich auch zum Offenbarungsgehalt der vorliegenden Anmeldung. Die Herstellung weiterer erfindungsgemäßer Verbindungen kann analog zu den oben gezeigten beispielhaften Reaktionen erfolgen oder nach anderen dem Fachmann an sich aus der Literatur bekannten Methoden erfolgen.

Vorteile der erfindungsgemäßen Verbindungen, bevorzugt der Formel (II), insbesondere der Formeln (IIa) bis (IId), können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),
- nach einfachen Verfahren herstellbar,
- schwache Schaumwirkung und/oder geringe Schaumstabilisierung,
- gute Verarbeitbarkeit in Formulierungen und/oder
- Lagerstabilität.

Bevorzugt können die erfindungsgemäßen Verbindungen eine besondere Oberflächenaktivität aufweisen. Die erfindungsgemäßen Verbindungen der Formel (I), insbesondere die Verbindungen der Formel (II), bevorzugt der Formeln (II-a) (II-b), (II-c) und (II-d), können gegenüber den Fluortensiden des Standes der Technik deutlich verbesserte Umwelteigenschaften aufweisen, da sie weder chemisch noch biologisch zu langkettigen PFCAs oder PFASs abbauen.

Bevorzugt können die erfindungsgemäßen Verbindungen durch entsprechende Umwelteinflüsse vollständig in mineralisierbare/regenerierbare Verbindungen überführt werden.

Der Abbau der erfindungsgemäßen Verbindungen kann bevorzugt nach zwei Mechanismen erfolgen. Im ersten Schritt kann durch biologische Aktivität das Kohlenstoffgerüst soweit abgebaut werden, dass (teil)fluorierte Verbindungen entstehen, die nicht toxisch sind und einen hohen Dampfdruck besitzen (Sdp <140°C). Aufgrund der hohen Flüchtigkeit, gelangen können diese Verbindungen in die Atmosphäre gelangen und in der Stratosphäre durch die dort vorherrschende intensive UV-Strahlung zu niedermolekularen Verbindungen (HF, $COF_2$ etc.) zersetzt werden. Diese Zersetzungsprodukte können dann mit dem Regen aus der Atmosphäre ausgewaschen, in den Boden überführt und dort mineralisiert werden.

Um diesen Zyklus durchlaufen zu können ist es bevorzugt, dass die Endprodukte bei der biologischen Zersetzung nicht perfluoriert sind und keine (stabilen) Salze entstehen können.

Bevorzugt kann die Alkylsubstitution am α-Kohlenstoff dazu führen, dass diese sekundären bzw. tertiären Alkohole beim biologischen Abbau vorrangig nicht zu schwer flüchtigen Verbindungen, wie z. B. Carbonsäuren, oxidiert werden.

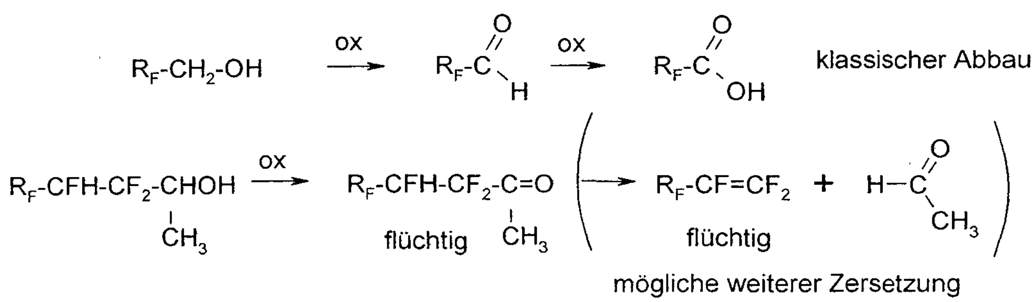


**Meta 1** **Meta 2**

Der nicht fluorierte Teil der erfindungsgemäßen Verbindungen ist über biologische Mechanismen leicht abbaubar. In allen Fällen verbleiben als biologisch nicht verwertbare Reste die Moleküle Meta 1 oder Meta 2, die einen hinreichend hohen Dampfdruck besitzen um aus dem Wasser oder Boden in die Atmosphäre gelangen zu können. Literaturangaben zufolge wird Meta 2 innerhalb kurzer Zeit in der Stratosphäre durch UV-Strahlung zersetzt (siehe

"Atmospheric Fate of various fluorocarbons, Master Thesis Yi Tang, Massachusetts Institute of Technology, 1993).

Ein weiterer Gegenstand der Erfindung ist ein Verfahren zum Abbau von fluorhaltigen Verbindungen umfassend die folgenden Schritte:

a) biologische und/oder abiotischer Abbau des Kohlenstoffgerüsts der fluorhaltigen Verbindungen unter Bildung von, vorzugsweise nicht toxischen, fluorhaltigen Verbindungen mit einem ausreichend hohen Dampfdruck,
b) Überführen der in Schritt a) gebildeten fluorhaltigen Verbindungen mit hohem Dampfdruck in eine Gasphase,
c) Abbau der in Schritt a) gebildeten fluorhaltigen Verbindungen mit hohem Dampfdruck zu niedermolekularen Verbindungen durch UV-Bestrahlung in der Gasphase,
d) Überführen der in Schritt c) gebildeten niedermolekularen Verbindungen aus der Gasphase in eine flüssige und/oder feste Phase,
e) Mineralisierung der Schritt c) gebildeten niedermolekularen Verbindungen der flüssigen und/oder festen Phase.

Bevorzugt werden in Schritt a) keine fluorhaltigen, Salze gebildet. Insbesondere werden in Schritt a) keine perfluorierten Verbindungen gebildet.

Bevorzugt werden fluorhaltige Tenside dem beschriebenen Abbauverfahren unterzogen, insbesondere solche Tenside, die auf teilfluorierten und/oder $\alpha$-alkylierten Alkoholen beruhen.

In dem genannten Abbauverfahren werden bevorzugt Verbindungen der Formel (I) eingesetzt, insbesondere solche der Formel (II), bevorzugt solche der Formeln (II-a) (II-b), (II-c) und (II-d).

Bevorzugt können in dem Abbauverfahren in Schritt a) Verbindungen der Formel (XV) und/oder der Formel (XVI) gebildet werden:

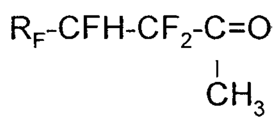

Formel (XV)

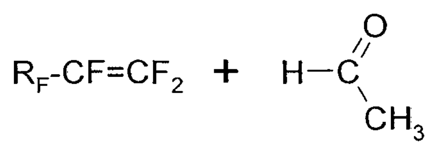

Formel (XVI)

Ein zweiter Gegenstand der vorliegenden Erfindung ist die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen. Fluortenside der Formeln (II) bis (XI) werden bevorzugt verwendet, insbesondere die genannten besonders bevorzugten Verbindungen, bevorzugt Verbindungen der Formeln (II-a) (II-b), (II-c) und (II-d).

Neben den Verbindungen der Formel (I), bevorzugt der Formel (II), insbesondere der Formeln (II-a) (II-b), (II-c) und (II-d), können die erfindungsgemäßen Mischungen auch Lösemittel, Additive, Hilfs- und Füllstoffe sowie nicht fluorierte Tenside enthalten. Beispielhaft seien genannt Silikonpartikel, Weichmacher und oberflächenmodifizierte Pigmente.

Bevorzugte Einsatzgebiete sind beispielsweise die Verwendung der erfindungsgemäßen Fluortenside, bevorzugt solche der Formel (II), insbesondere der Formeln (II-a) (II-b), (II-c) und (II-d), als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen, in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen.

Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung. Entsprechende Mittel, enthaltend die erfindungsgemäßen Fluortensiden,

sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Lackformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die vollständigen Offenbarungen aller aufgeführten Anmeldungen und Veröffentlichungen sind durch Bezugnahme in diese Anmeldung eingeführt. Für die vorliegende Erfindung bedeutet sowohl die Pluralform eines Begriffs als auch die Singularform eines Begriffs auch die jeweils andere Form, soweit nicht ausdrücklich anderes angegeben ist. Alle Merkmale der vorliegenden Erfindung können in jeder Art miteinander kombiniert werden, es sei denn dass sich bestimmte Merkmale ausschließen. Dies gilt vor allem für bevorzugte und besonders bevorzugte Merkmale. Weitere Merkmale, Vorteile und Varianten der Erfindung ergeben sich auch aus den Ansprüchen und Beispielen. Die folgenden Beispiele erläutern die vorliegende Erfindung näher, ohne den Schutzbereich zu beschränken.

## Beispiele

### Abkürzungen

MTBE tert-Butylmethylether
Sdp. Siedepunkt
VE vollentsalzt

### Beispiel 1:

2-Sulfo-succinic acid bis-[2-(2,2,3-trifluoro-3-heptafluoropropyloxy-propoxy)-ethyl] ester sodium

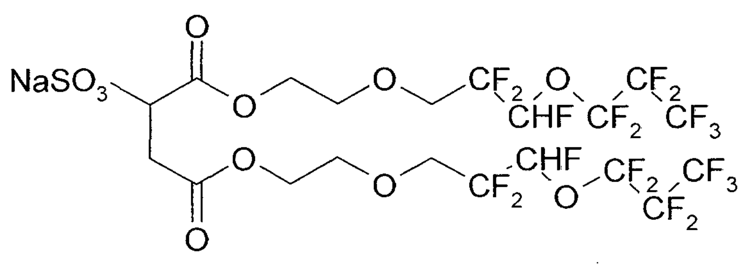

20,20 g 2,2,3-Trifluoro-3-heptafluoropropyloxy-propan-1-ol, 6,56 g Ethylencarbonat und 0,95 g Kaliumcarbonat

werden 75 Stunden bei 130°C zur Reaktion gebracht. Das Reaktionsgemisch wird mit 50 mL halbkonzentrierter HCl versetzte und mit 3x 25 ml MTBE ausgeschüttelt, die vereinigten organischen Phasen werden über Natriumsulfat getrocknet und das Lösemittel abdestilliert. Rohausbeute: 21,75 g

Nach destillativer Aufarbeitung erhält mal 12,9 g 96% Produkt (Sdp 78°C/ 10 mbar).

GC -MS und NMR Untersuchungen bestätigen die Struktur

12,65 g des Alkohols, 1,72 g Maleinsäureanhydrid und 0,3g p-Toluolsulfonsäure (Katalysator) in 60 ml Toluol werde unter Rückfluss 96 Stunden gekocht. Das bei der Reaktion freiwerdende Wasser wird mit Hilfe des Wasserabscheiders entfernt. Das Reaktionsgemisch wird mit 30 mL VE-Wasser versetzt und die Phasen getrennt. Anschließend wird die wässrige Phase mit 3 x 25 mL MTBE extrahiert, die vereinigten organischen Phasen mit 1 x 30 mL Wasser und 1 x 30 mL ges. NaCl-Lösung gewaschen und über Natriumsulfat getrocknet, abfiltriert und das Lösungsmittel abdestilliert.

Nach Destillation im Hochvakuum erhält man 8,22 g des entsprechenden Esters in 96 %iger Reinheit (Sdp. 180°/ 6,7*$10^{-2}$ mbar).

6,6 g Diesters werden in 12 ml 2-Propanol gelöst und bei 50°C mit 5,57g 39% wässriger Natriumhydrogensulfitlösung versetzt .Der pH wird mit 32%ige Natronlauge auf 5-6 eingestellt und 41 h bei 100 °C gerührt.

Das Reaktionsgemisch wird mit 40 mL VE-Wasser versetzt und die Phasen getrennt.

Anschließend wird die wässrige Phase mit 3 x 25 mL MTBE extrahiert, die vereinigten organischen Phasen mit 1 x 30 mL Wasser und 1 x 30 mL gesättigter NaCl-Lösung gewaschen und über Natriumsulfat getrocknet. Nach Abdestillation des Lösungsmittel erhält man 3,90 g Ausbeute

Die statische Oberflächenspannung $\gamma_{stat}$. wird nach der angegebenen Methode bestimmt und beträgt 17,69 mN/m für eine Lösung mit 0.1 Gew.-% der Verbindung und 16,78 mN/m für eine Lösung mit 1 Gew.-% der Verbindung.

Die dynamische Oberflächenspannung ist in Abbildung 1 wiedergegeben.

**Beispiel 2: Bestimmung der statischen Oberflächenspannung**

Es werden die statischen Oberflächenspannungen $\gamma$ von wässrigen Tensidlösungen mit verschiedenen Konzentrationen c (Gramm pro Liter) bestimmt.

Gerät: Tensiometer der Firma Dataphysics (Modell DCAT 11)
Temperatur der Messlösungen: 20°±0,2°C
Eingesetzte Messmethode: Messung der Oberflächenspannung mit der Wilhelmy Plattenmethode nach DIN EN 14370.
Platte: Platin, Länge= 19,9mm

Bei der Plattenmethode wird die Oberflächen- bzw. Grenzflächenspannung der Tensidlösung aus der auf die benetzte Länge einer Platte wirkenden Kraft nach folgender Formel berechnet.

$$\gamma = \frac{F}{L \cdot \cos\theta} = \frac{F}{L}$$

$\gamma$= Grenz- oder Oberflächenspannung; F= auf die Waage wirkende Kraft;
L= benetzte Länge (19,9 mm); $\theta$= Kontaktwinkel)

Die Platte besteht aus angerautem Platin und wird also optimal benetzt, so dass der Kontaktwinkel $\theta$ nahe bei 0° liegt. Der Term cos $\theta$ erreicht daher annähernd den Wert 1, so dass nur noch die gemessene Kraft und die Länge der Platte berücksichtigt werden müssen.

**Beispiel 3: Bestimmung der dynamischen Oberflächenspannung**

Es wird die dynamische Oberflächenspannung $\gamma$ einer 0.1%igen (Gewichtsprozent) wässrigen Lösung der zu untersuchenden Verbindung bestimmt.

Eingesetzte Messmethode: Messung der Oberflächenspannung mit der Blasendruckmethode
Gerät: Tensiometer der Firma SITA (Modell t 60)
Temperatur der Messlösungen: 20°C ± 0,2°C

Bei der Messung der dynamischen Oberflächenspannung werden Luftblasen mit verschiedenen Geschwindigkeiten durch eine Kapillare in die Tensidlösung gedrückt. Aus der dabei auftretenden Druckänderung kann die Oberflächenspannung in Abhängigkeit von der Blasenlebensdauer mit folgender Gleichung bestimmt werden:

$$\gamma = \frac{r(p_{max} - \rho \cdot g \cdot h)}{2}$$

$p_{max}$= Maximaldruck, $\rho$ = Dichte der Flüssigkeit, h= Eintauchtiefe, r= Radius der Kapillare

**Patentansprüche**

1. Verbindungen der Formel (I) oder (I')

$Z_nC_cD_dSpacer_mX_x$ (I)

$(ZC_cD_d)_{n'}Spacer_mX_x$ (I')

wobei

Z = $R_f$-O-CHF-$CF_2$-$CR^1R^2$-O- oder
$R_f$-O-$(CF_2)_{1-4}$-CHF-$CF_2$-$CR^1R^2$-O- oder
$R_f$-O-$(CF_2)_{1-4}$-O-CHF-$CF_2$-$CR^1R^2$-O- oder
$R_f$-O-$(CF(CF_3)$-$CF_2)_{1-4}$-CHF-$CF_2$-$CR^1R^2$-O- oder
$R_f$-O-$(CF(CF_3)$-$CF_2)_{1-4}$-O-CHF-$CF_2$-$CR^1R^2$-O- oder
$R_f$-O-$(CF_2$-$O)_{1-4}$-CHF-$CF_2$-$CR^1R^2$-O- oder
$R_f$-O-$(CF_2$-$CF_2$-$O)_{1-4}$-CHF-$CF_2$-$CR^1R^2$-O- oder
$R_f$-O-$(CF_2$-$O)_{1-4}$-$(CF_2$-$CF_2$-$O)_{1-4}$-CHF-$CF_2$-$CR^1R^2$-O-
n und n' = 1, 2, 3,4, 5 oder 6,
$R_f$ = fluoriertes Alkyl, linear oder verzweigt,
$R^1$ = H oder Alkyl, bevorzugt C1-C4, insbesondere $-CH_3$ oder $-CH_2$-$CH_3$
$R^2$ = H oder Alkyl, bevorzugt C1-C4, insbesondere $-CH_3$,
C = $-CR'_2$-$CR''_2$-O-,
c = 0 oder eine ganze Zahl aus dem Bereich von 1 bis 100,
R' und R" = unabhängig voneinander H oder Alkyl,
D = -CO-
d = 0 oder 1,
Spacer = eine gesättigte oder ungesättigte, verzweigte oder unverzweigte, ggf. Heteroatome enthaltende, Kohlenwasserstoffeinheit,
m = 0 oder 1,
X eine anionische, kationische, nicht-ionische oder amphotere hydrophile Gruppe ist ausgewählt aus den Gruppen $-COO^-$, $-SO_3^-$, $-OSO_3^-$, $-PO_3^{2-}$, $-OPO_3^{2-}$, $-(OCH_2CH_2)_s$-O-$(CH_2)_t$-$COO^-$, $-(OCH_2CH_2)_s$-O-$(CH_2)_t$-$SO_3^-$, $-(OCH_2CH_2)_s$-O-$(CH_2)_t$-$OSO_3^-$, $-(OCH_2CH_2)_s$-O-$(CH_2)_t$-$PO_3^{2-}$, $-(OCH_2CH_2)_s$-O-$(CH_2)_t$-$OPO_3^{2-}$, den Formeln A bis C,

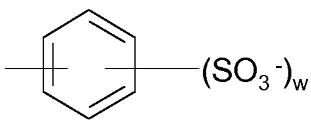

A

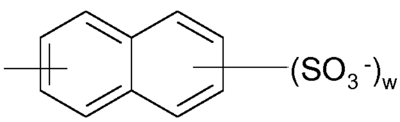

B

oder

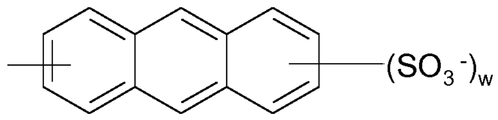

C

wobei s steht für eine ganze Zahl aus dem Bereich von 1 bis 1000, t steht für eine ganze Zahl ausgewählt aus 1, 2, 3 oder 4 und w steht für eine ganze Zahl ausgewählt aus 1, 2 oder 3,

oder

$-NR^1R^2R^{3+}$ $Z^-$, $-PR^1R^2R^{3+}$ $Z^-$,

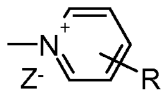 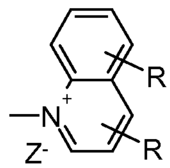 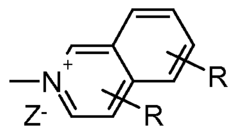

wobei R steht für H oder $C_{1-4}$-Alkyl in beliebiger Position,

$Z^-$ steht für $Cl^-$, $Br^-$, $I^-$, $CH_3SO_3^-$, $CF_3SO_3^-$, $CH_3PhSO_3^-$, $PhSO_3^-$

$R^1$, $R^2$ und $R^3$ jeweils unabhängig voneinander stehen für H, $C_{1-30}$-Alkyl, Ar oder $-CH_2Ar$ und

Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können,

oder

lineares oder verzweigtes Alkyl, wobei ein oder mehrere nicht benachbarte C-Atome durch O, S und/oder N ersetzt sind, -OH, -SH, -O-(Glycosid)$_{o'}$, -S-(Glycosid)$_{o'}$, $-OCH_2-CHOH-CH_2-O$ H, $-OCH_2Ar(-NCO)_{p'}$, $-OAr(-NCO)_{p'}$, Aminoxid,

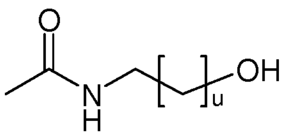 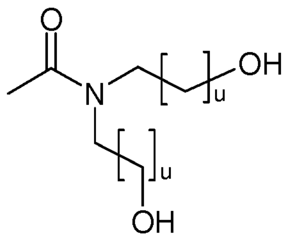 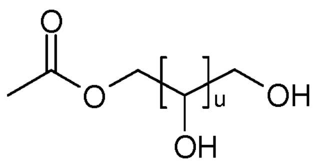

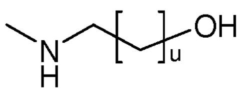 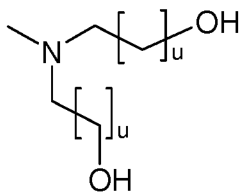 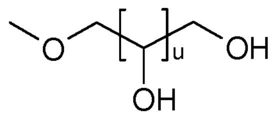

u steht für eine ganze Zahl aus dem Bereich von 1 bis 6, bevorzugt 1 bis 4,

o' steht für eine ganze Zahl aus dem Bereich von 1 bis 10,

p' steht für 1 oder 2,

Ar steht für einen unsubstituierten, ein- oder mehrfach substituierten aromatischen Ring oder kondensierte Ringsysteme mit 6 bis 18 C-Atomen, worin auch ein oder zwei CH-Gruppen durch C=O ersetzt sein können und,

Glycosid steht für ein verethertes Kohlenhydrat, vorzugsweise für ein mono-, di-, tri- oder oligo-Glucosid,

oder

Acetyldiamine, N-Alkylaminosäuren, N-Alkylaminosulfonsäuren, Betaine, Sulfobetaine,

und x = 1, 2, 3 oder 4 ist.

**2.** Verbindungen gemäß Anspruch 1, **dadurch gekennzeichnet, dass** sie der Formel (I') entsprechen.

**3.** Verbindungen gemäß einem oder mehreren der Ansprüche 1 bis 2, **dadurch gekennzeichnet, dass** n gleich 2-3 ist.

**4.** Verbindungen gemäß einem oder mehreren der Ansprüche 1 bis 3, **dadurch gekennzeichnet, dass** $R_f$ = fluoriertes C1-C6-Alkyl, insbesondere fluoriertes C1-C3-Alkyl, ist.

**5.** Verbindungen gemäß einem oder mehreren der Ansprüche 1 bis 4, **dadurch gekennzeichnet, dass** x = 1.

**6.** Verbindungen gemäß einem oder mehreren der Ansprüche 1 bis 5, **dadurch gekennzeichnet, dass** Z gleich: $R_f$-O-CHF-$CF_2$-$CR^1R^2$-O- oder $R_f$-O-$(CF_2)_{1-4}$-CHF-$CF_2$-$CR^1R^2$-O- ist mit $R^1$ und $R^2$ unabhängig voneinander = H, -$CH_3$ oder -$CH_2$-$CH_3$.

**7.** Verbindungen einem oder mehreren der Ansprüche 1 bis 6, **dadurch gekennzeichnet, dass** Rf = $CF_3$- oder $CF_3$-$CF_2$- oder $CF_3$-$CF_2$-$CF_2$-ist.

**8.** Verbindungen gemäß einem oder mehreren der Ansprüche 1 bis 7, **dadurch gekennzeichnet, dass** sie den Formeln (II) bis (XI) entsprechen mit

Y = $-(OCR''_2-CR'_2)_{0/1}-Z$,

X = $Na^+$, $K^+$, $Li^+$, $NH^{4+}$, $N(CH_3)^{4+}$,

Q = Cl, Br, I

und R' und R" = unabhängig voneinander H oder C1-C4 Alkyl

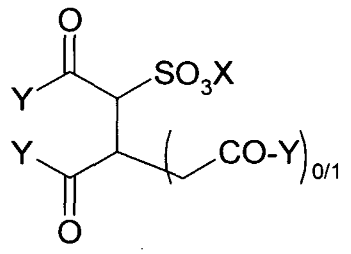

Formel (II)

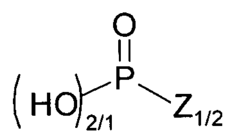

Formel (III)

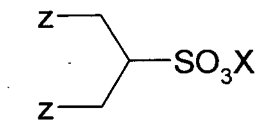

Formel (IV)

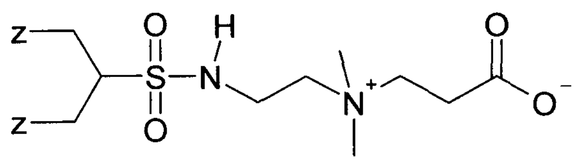

Formel (V)

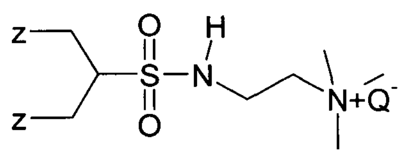

Formel (VI)

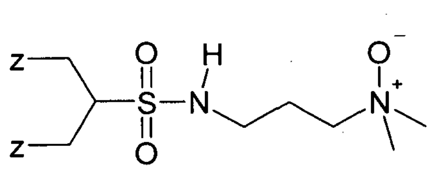

Formel (VII)

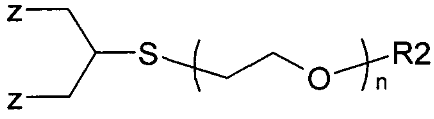

Formel (VIII)

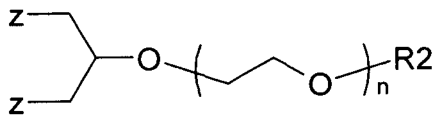

Formel (IX)

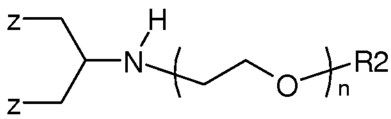

Formel (X)

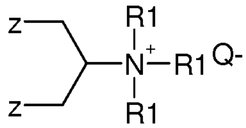

Formel (XI)

**9.** Verbindungen einem oder mehreren der Ansprüche 1 bis 8, **dadurch gekennzeichnet, dass** Y = $-(OCH_2-CHR'_2)_{0/1}-Z$, mit R' = H oder C1-C2 Alkyl.

**10.** Verbindungen gemäß einem oder mehreren der Ansprüche 1 bis 9, **dadurch gekennzeichnet, dass** sie den Formeln (II-a), (II-b), (II-c) oder (II-d) entsprechen:

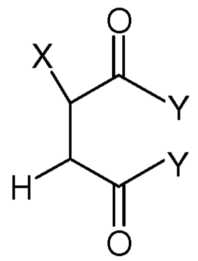

(II-a)

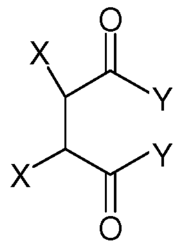

(II-b)

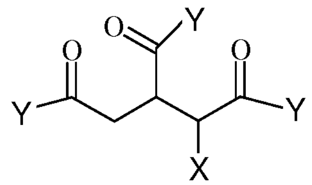

(II-c)

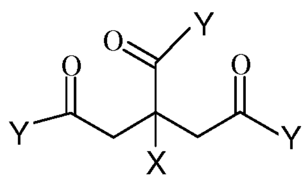

(II-d)

**11.** Verbindungen gemäß einem oder mehreren der Ansprüche 1 bis 10, **dadurch gekennzeichnet, dass** Rf = $CF_3$-, $CF_3$-$CF_2$- oder $CF_3$-$CF_2$-$CF_2$-, Z = $R_f$-O-CHF-$CF_2$-$CR^1R^2$-O- oder $R_f$-O-$(CF_2)_{1-4}$-CHF-$CF_2$-$CR^1R^2$-O-, mit $R^1$ und

$R^2$ unabhängig voneinander = H, $-CH_3$ oder-$CH_2$-$CH_3$ und X eine der Gruppen $-SO_3^-$, $-OSO_3^-$, $-PO_3^{2-}$ oder $OPO_3^{2-}$, Polyethylen- oder Polypropylenglykole, -CH(OH)-$CH_2$-NH-Sach,-Y-($CH_2$-$CH_2$-O)$_v$-$R^4$, Betaine oder Sulfobetaine ist, mit Sach = verschiedene Zucker, Y = S, O oder NH, $R^4$ = H oder Alkyl und v = 1-100.

**12.** Verwendung von Verbindungen gemäß einem oder mehreren der Ansprüche 1 bis 11 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**13.** Mittel enthaltend eine Verbindung gemäß einem oder mehreren der Ansprüche 1 bis 11 und einen für den jeweiligen Verwendungszweck geeigneten Träger sowie ggf. weitere spezifische Aktivstoffe.

**14.** Mittel nach Anspruch 13, **dadurch gekennzeichnet, dass** es sich bei dem Mittel um Farb- und Lackzubereitungen, Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

## Claims

**1.** Compounds of the formula (I) or (I')

$$Z_nC_cD_dSpacer_mX_x \quad (I)$$

$$(ZC_cD_d)_nSpacer_mX_x \quad (I')$$

where

Z = $R_f$-O-CHF-$CF_2$-$CR^1R^2$-O- or
$R_f$-O-($CF_2$)$_{1-4}$-CHF-$CF_2$-$CR^1R^2$-O- or
$R_f$-O-($CF_2$)$_{1-4}$-O-CHF-$CF_2$-$CR^1R^2$-O- or
$R_f$-O-(CF($CF_3$)-$CF_2$)$_{1-4}$-CHF-$CF_2$-$CR^1R^2$-O- or
$R_f$-O-(CF($CF_3$)-$CF_2$)$_{1-4}$-O-CHF-$CF_2$-$CR^1R^2$-O- or
$R_f$-O-($CF_2$-O)$_{1-4}$-CHF-$CF_2$-$CR^1R^2$-O- or
$R_f$-O-($CF_2$-$CF_2$-O)$_{1-4}$-CHF-$CF_2$-$CR^1R^2$-O- or
$R_f$-O-($CF_2$-O)$_{1-4}$-($CF_2$-$CF_2$-O)$_{1-4}$-CHF-$CF_2$-$CR^1R^2$-O-.
n and n' = 1, 2, 3, 4, 5 or 6,
$R_f$= fluorinated alkyl, linear or branched,
$R^1$ = H or alkyl, preferably C1-C4, in particular $-CH_3$ or $-CH_2$-$CH_3$,
$R^2$ = H or alkyl, preferably C1-C4, in particular $-CH_3$,
C = -$CR'_2$-$CR''_2$-O-,
c = 0 or an integer from the range from 1 to 100,
R' and R" = independently of one another H or alkyl,
D = -CO-,
d = 0 or 1,
spacer = a saturated or unsaturated, branched or unbranched hydrocarbon unit, optionally containing heteroatoms,
m = 0 or 1,
X an anionic, cationic, nonionic or amphoteric hydrophilic selected from the groups
$-COO^-$, $-SO_3^-$, $-OSO_3^-$, $-PO_3^{2-}$, $-OPO_3^{2-}$, -($OCH_2CH_2$)$_s$-O-($CH_2$)$_t$-$COO^-$,
-($OCH_2CH_2$)$_s$-O-($CH_2$)$_t$-$SO_3^-$, -($OCH_2CH_2$)$_s$-O-($CH_2$)$_t$-$OSO_3^-$,
-($OCH_2CH_2$)$_s$-O-($CH_2$)$_t$-$PO_3^{2-}$, -($OCH_2CH_2$)$_s$-O-($CH_2$)$_t$-$OPO_3^{2-}$, the formulae A to C,

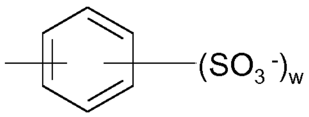

A

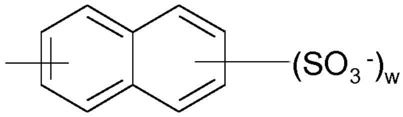

B or

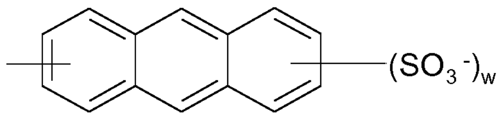

C

where s stands for an integer from the range from 1 to 1000, t stands for an integer selected from 1, 2, 3 or 4 and w stands for an integer selected from 1, 2 or 3,

or

$-NR^1R^2R^{3+} Z^-$, $-PR^1R^2R^{3+} Z^-$,

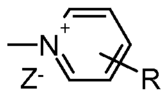
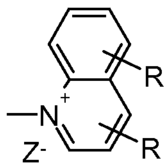
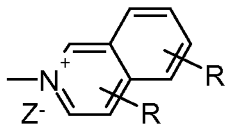

where R stands for H or $C_{1-4}$-alkyl in any desired position,

$Z^-$ stands for $Cl^-$, $Br^-$, $I^-$, $CH_3SO_3^-$, $CF_3SO_3^-$, $CH_3PhSO_3^-$, $PhSO_3^-$

$R^1$, $R^2$ and $R^3$ in each case, independently of one another, stand for H, $C_{1-30}$-alkyl, Ar or $-CH_2Ar$ and

Ar stands for an unsubstituted or mono- or polysubstituted aromatic ring or condensed ring systems having 6 to 18 C atoms, in which, in addition, one or two CH groups may be replaced by N,

or

linear or branched alkyl, in which one or more non-adjacent C atoms are replaced by O, S, and/or N,

-OH, -SH, -O-(glycoside)$_{o'}$, -S-(glycoside)$_{o'}$, $-OCH_2-CHOH-CH_2-OH$, $-OCH_2Ar(-NCO)_{p'}$, $-OAr(-NCO)_{p'}$, amine oxide,

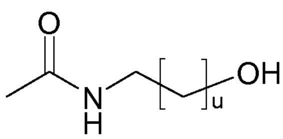
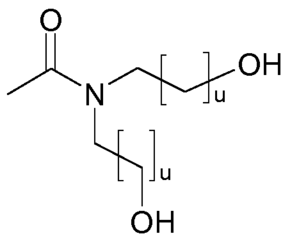
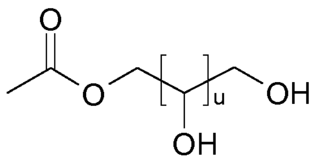

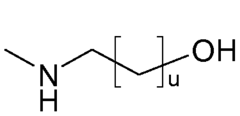
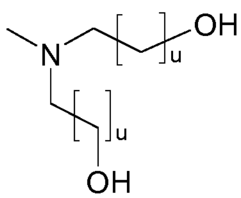
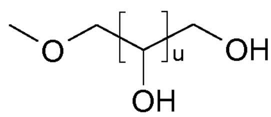

u stands for an integer from the range from 1 to 6, preferably 1 to 4,

o' stands for an integer from the range from 1 to 10,

p' stands for 1 or 2,

Ar stands for an unsubstituted, mono- or polysubstituted aromatic ring or condensed ring systems having 6 to 18 C atoms, in which, in addition, one or two CH groups may be replaced by C=O and, glycoside stands for an etherified carbohydrate, preferably for a mono-, di-, tri- or oligoglucoside,

or

acetyldiamines, N-alkylamino acids, N-alkylaminosulfonic acids, betaines, sulfobetaines,

and x = 1, 2, 3 or 4.

2. Compounds according to Claim 1, **characterised in that** they conform to the formula (I').

3. Compounds according to one or more of Claims 1 to 2, **characterised in that** n is equal to 2-3.

4. Compounds according to one or more of Claims 1 to 3, **characterised in that** $R_f$ = fluorinated C1-C6-alkyl, in particular fluorinated C1-C3-alkyl.

5. Compounds according to one or more of Claims 1 to 4, **characterised in that** x = 1.

6. Compounds according to one or more of Claims 1 to 5, **characterised in that** Z is equal to:

$$R_f\text{-O-CHF-CF}_2\text{-CR}^1\text{R}^2\text{-O- or } R_f\text{-O-(CF}_2)_{1\text{-}4}\text{-CHF-CF}_2\text{-CR}^1\text{R}^2\text{-O-,}$$

where $R^1$ and $R^2$ independently of one another = H, $-CH_3$ or $-CH_2-CH_3$.

7. Compounds according to one or more of Claims 1 to 6, **characterised in that** Rf = $CF_3$- or $CF_3$-$CF_2$- or $CF_3$-$CF_2$-$CF_2$-.

8. Compounds according to one or more of Claims 1 to 7, **characterised in that** they conform to the formulae (II) to (XI), where

$Y = -(OCH_2\text{-}CHR'_2)_{0/1}\text{-}Z$,

$X = Na^+, K^+, Li^+, NH^{4+}, N(CH_3)^{4+}$,

Q = Cl, Br, I

and R' and R" = independently of one another H or C1-C4 alkyl,

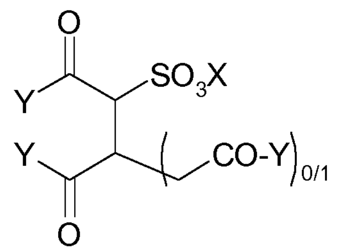

Formula (II)

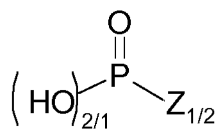

Formula (III)

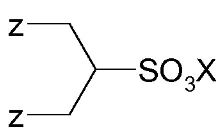

Formula (IV)

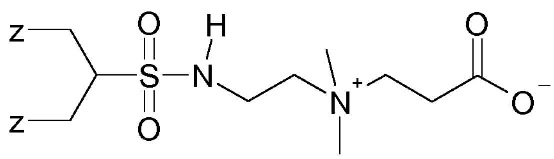

Formula (V)

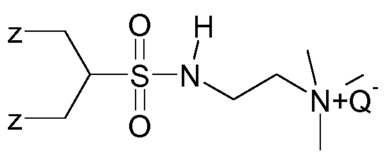

Formula (VI)

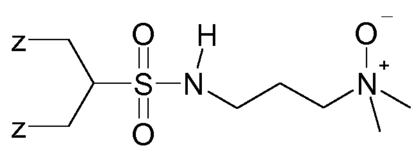

Formula (VII)

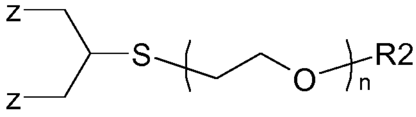

Formula (VIII)

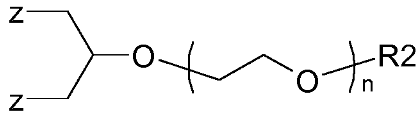

Formula (IX)

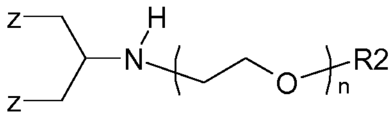

Formula (X)

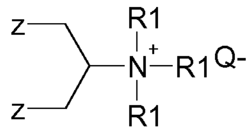

Formula (XI)

**9.** Compounds according to one or more of Claims 1 to 8, **characterised in that** Y = $-(OCH_2-CHR'_2)_{0/1}-Z$, where R' = H or C1-C2 alkyl.

**10.** Compounds according to one or more of Claims 1 to 9, **characterised in that** they conform to the formulae (II-a), (II-b), (II-c) or (II-d):

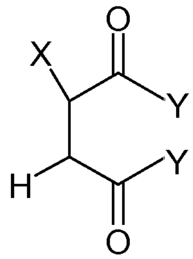

(II-a)

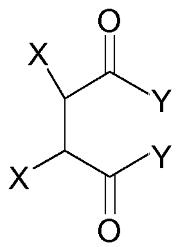

(II-b)

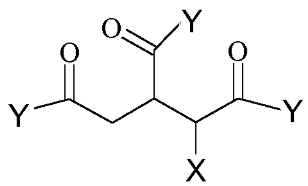

(II-c)

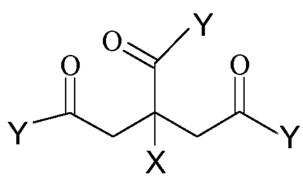

(II-d)

**11.** Compounds according to one or more of Claims 1 to 10, **characterised in that** Rf = $CF_3$-, $CF_3-CF_2$- or $CF_3-CF_2-CF_2$-, Z = $R_f-O-CHF-CF_2-CR^1R^2-O-$ or $R_f-O-(CF_2)_{1-4}-CHF-CF_2-CR^1R^2-O-$, where $R^1$ and $R^2$ independently of one another = H, $-CH_3$ or $-CH_2-CH_3$ and X is one of the groups $-SO_3^-$, $-OSO_3^-$, $-PO_3^{2-}$ or $OPO_3^{2-}$, polyethylene glycols or

polypropylene glycols, $-CH(OH)-CH_2-NH-sach$, $-Y-(CH_2-CH_2-O)_v-R^4$, betaines or sulfobetaines, where sach = various sugars, Y = S, O or NH, $R^4$ = H or alkyl and v = 1=100.

12. Use of compounds according to one or more of Claims 1 to 11 as additives in paints, coatings, printing inks, protective coatings, speciality coatings in electronic or optical applications, photoresists, top antireflective coatings or bottom antireflective coatings, developer solutions and wash solutions and photoresists for photolithographic processes, cosmetic products, agrochemicals, floor polishes, photographic coatings or coatings of optical elements.

13. Composition comprising a compound according to one or more of Claims 1 to 11 and a vehicle which is suitable for the respective application and optionally further specific active substances.

14. Composition according to Claim 13, **characterised in that** the composition encompasses paint and coating preparations, fire-extinguishing compositions, lubricants, washing and cleaning compositions, deicers, developer solutions and wash solutions and photoresists for photolithographic processes, cosmetic products, agrochemicals, floor polishes or hydrophobicising compositions for textile finishing or glass treatment.

## Revendications

1. Composés de formule (I) ou (I')

$$Z_nC_cD_d\text{espaceur}_mX_x \qquad (I)$$

$$(ZC_cD_d)_n\text{espaceur}_mX_x \qquad (I')$$

où

Z = $R_f$-O-CHF-$CF_2$-$CR^1R^2$-O- ou
$R_f$-O-$(CF_2)_{1-4}$-CHF-$CF_2$-$CR^1R^2$-O- ou
$R_f$-O-$(CF_2)_{1-4}$-O-CHF-$CF_2$-$CR^1R^2$-O- ou
$R_f$-O-$(CF(CF_3)-CF_2)_{1-4}$-CHF-$CF_2$-$CR^1R^2$-O- ou
$R_f$-O-$(CF(CF_3)-CF_2)_{1-4}$-O-CHF-$CF_2$-$CR^1R^2$-O- ou
$R_f$-O-$(CF_2-O)_{1-4}$-CHF-$CF_2$-$CR^1R^2$-O- ou
$R_f$-O-$(CF_2-CF_2-O)_{1-4}$-CHF-$CF_2$-$CR^1R^2$-O- ou
$R_f$-O-$(CF_2-O)_{1-4}$-$(CF_2-CF_2-O)_{1-4}$-CHF-$CF_2$-$CR^1R^2$-O-.
n et n' = 1, 2, 3, 4, 5 ou 6,
$R_f$ = alkyle fluoré, linéaire ou ramifié,
$R^1$ = H ou alkyle, de préférence en C1-C4, en particulier $-CH_3$ ou $-CH_2-CH_3$,
$R^2$ = H ou alkyle, de préférence en C1-C4, en particulier $-CH_3$,
C = $-CR'_2-CR''_2-O-$,
c = 0 ou un nombre entier dans la plage allant de 1 à 100,
R' et R" = indépendamment l'un de l'autre H ou alkyle,
D = -CO-,
d = 0 ou 1,
espaceur = un motif hydrocarboné saturé ou insaturé, ramifié ou non ramifié, contenant éventuellement des hétéroatomes,
m = 0 ou 1,
X est un groupement hydrophile anionique, cationique, non ionique ou amphotère, choisi parmi les groupements $-COO^-$, $-SO_3^-$, $-OSO_3^-$, $-PO_3^{2-}$, $-OPO_3^{2-}$, $-(OCH_2CH_2)_s-O-(CH_2)_t-COO^-$, $-(OCH_2CH_2)_s-O-(CH_2)_t-SO_3^-$, $-(OCH_2CH_2)_s-O-(CH_2)_t-OSO_3^-$, $-(OCH_2CH_2)_s-O-(CH_2)_t-PO_3^{2-}$, $-(OCH_2CH_2)_s-O-(CH_2)_t-OPO_3^{2-}$ les formules A à C,

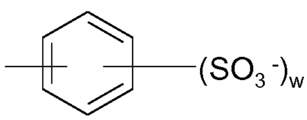
A

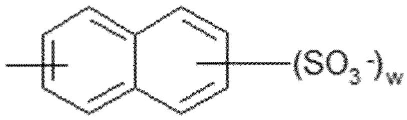

B ou

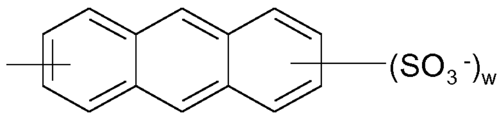

C

où s représente un nombre entier dans la plage allant de 1 à 1000, t représente un nombre entier choisi parmi 1, 2, 3 ou 4 et w représente un nombre entier choisi parmi 1, 2 ou 3,

ou

$-NR^1R^2R^{3+}\ Z^-$, $-PR^1R^2R^{3+}\ Z^-$,

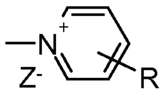 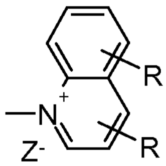 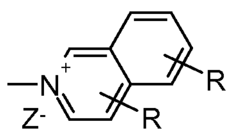

où R représente H ou $C_{1-4}$-alkyle dans une position désirée quelconque,

$Z^-$ représente $Cl^-$, $Br^-$, $I^-$, $CH_3SO_3^-$, $CF_3SO_3^-$, $CH_3PhSO_3^-$, $PhSO_3^-$

$R^1$, $R^2$ et $R^3$ dans chaque cas, indépendamment les uns des autres, représentent H, $C_{1-30}$-alkyle, Ar ou $-CH_2Ar$ et Ar représente un cycle aromatique non substitué ou mono- ou poly-substitué ou des noyaux condensés ayant de 6 à 18 atomes de C, où, de plus, un ou deux groupements CH peuvent être remplacés par N,

ou

alkyle linéaire ou ramifié, où un ou plusieurs atomes de C non adjacents sont remplacés par O, S, et/ou N, -OH, -SH, -O-(glycoside)$_{o'}$, -S-(glycoside)$_{o'}$, $-OCH_2-CHOH-CH_2-OH$, $-OCH_2Ar(-NCO)_{p'}$, $-OAr(-NCO)_{p'}$, oxyde d'amine,

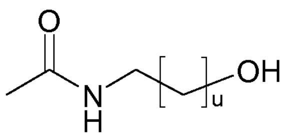 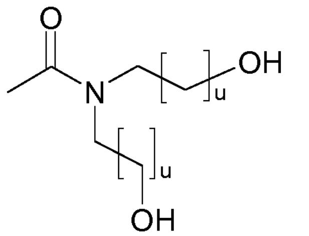 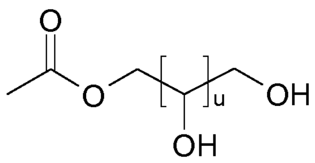

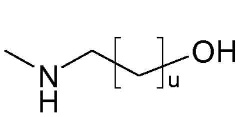 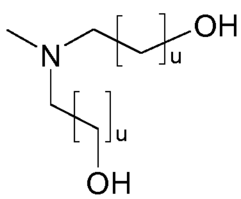 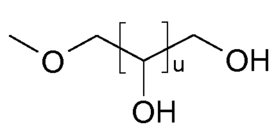

u représente un nombre entier dans la plage allant de 1 à 6, de préférence 1 à 4,

o' représente un nombre entier dans la plage allant de 1 à 10,

p' représente 1 ou 2,

Ar représente un cycle aromatique non substitué, mono- ou poly-substitué ou des noyaux condensés ayant de 6 à 18 atomes de C, où, de plus, un ou deux groupements CH peuvent être remplacés par C=O et,

glycoside représente un glucide éthérifié, de préférence un mono-, di-, tri- ou oligo-glucoside,

ou

des acétyldiamines, des acides N-alkylaminés, des acides N-alkyl-aminosulfoniques, des bétaïnes, des sulfobétaïnes,

et x = 1, 2, 3 ou 4.

**2.** Composés selon la revendication 1, **caractérisés en ce qu'**ils répondent à la formule (I').

**3.** Composés selon l'une ou plusieurs parmi les revendications 1 à 2, **caractérisés en ce que** n est égal à 2-3.

**4.** Composés selon l'une ou plusieurs parmi les revendications 1 à 3, **caractérisés en ce que** $R_f$ = C1-C6-alkyle fluoré, en particulier C1-C3-alkyle fluoré.

**5.** Composés selon l'une ou plusieurs parmi les revendications 1 à 4, **caractérisés en ce que** x = 1.

**6.** Composés selon l'une ou plusieurs parmi les revendications 1 à 5, **caractérisés en ce que** Z est égal à : $R_f$-O-CHF-$CF_2$-$CR^1R^2$-O- ou $R_f$-O-$(CF_2)_{1-4}$-CHF-$CF_2$-$CR^1R^2$-O-, où $R^1$ et $R^2$ indépendamment l'un de l'autre = H, -$CH_3$ ou -$CH_2$-$CH_3$.

**7.** Composés selon l'une ou plusieurs parmi les revendications 1 à 6, **caractérisés en ce que** Rf = $CF_3$- ou $CF_3$-$CF_2$- ou $CF_3$-$CF_2$-$CF_2$-.

**8.** Composés selon l'une ou plusieurs parmi les revendications 1 à 7, **caractérisés en ce qu'**ils répondent aux formules (II) à (XI), où

Y = -$(OCH_2\text{-}CHR'_2)_{0/1}$-Z,

X = $Na^+$, $K^+$, $Li^+$, $NH^{4+}$, $N(CH_3)^{4+}$,

Q = Cl, Br, I et

R' et R" = indépendamment l'un de l'autre H ou C1-C4 alkyle,

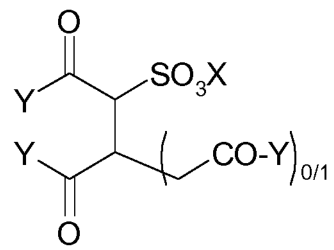

Formule (II)

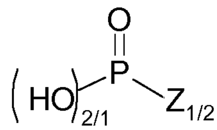

Formule (III)

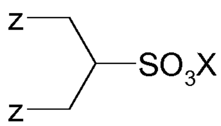

Formule (IV)

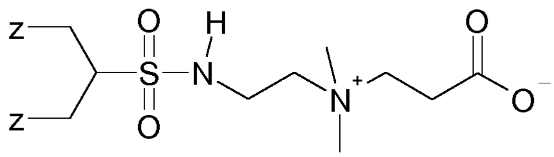

Formule (V)

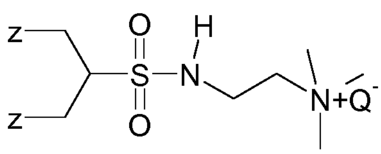

Formule (VI)

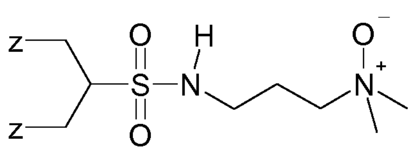

Formule (VII)

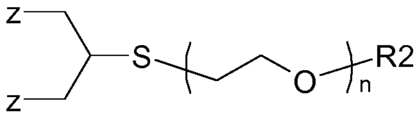

Formule (VIII)

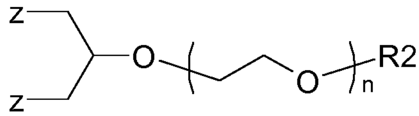

Formule (IX)

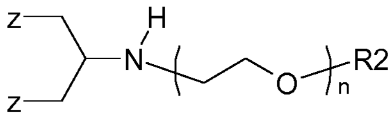

Formule (X)

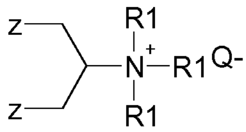

Formule (XI)

**9.** Composés selon l'une ou plusieurs parmi les revendications 1 à 8, **caractérisés en ce que** Y = $-(OCH_2-CHR'_2)_{0/1}-Z$, où R' = H ou C1-C2 alkyle.

**10.** Composés selon l'une ou plusieurs parmi les revendications 1 à 9, **caractérisés en ce qu'**ils répondent aux formules (II-a), (II-b), (II-c) ou (II-d):

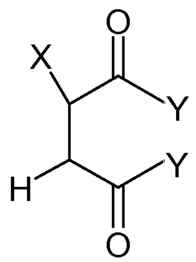

(II-a)

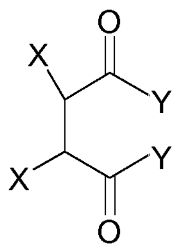

(II-b)

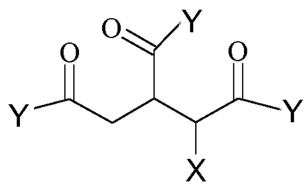

(II-c)

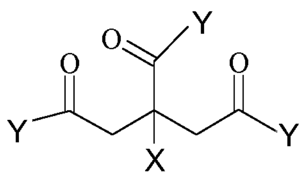

(II-d)

**11.** Composés selon l'une ou plusieurs parmi les revendications 1 à 10, **caractérisés en ce que** Rf = $CF_3$-, $CF_3$-$CF_2$- ou $CF_3$-$CF_2$-$CF_2$-, Z = $R_f$-O-CHF-$CF_2$-$CR^1R^2$-O- ou $R_f$-O-$(CF_2)_{1-4}$-CHF-$CF_2$-$CR^1R^2$-O-, où $R^1$ et $R^2$ indépendamment l'un de l'autre = H, $-CH_3$ ou $-CH_2-CH_3$ et X est l'un des groupements $-SO_3^-$, $-OSO_3^-$, $-PO_3^{2-}$ ou $OPO_3^{2-}$, des

polyéthylène glycols ou polypropylène glycols, $-CH(OH)-CH_2-NH-$« sach », $-Y-(CH_2-CH_2-O)_v-R^4$, des bétaïnes ou sulfobétaïnes, où « sach » = divers sucres, Y = S, O ou NH, $R^4$ = H ou alkyle et v = 1=100.

12. Utilisation de composés selon l'une ou plusieurs parmi les revendications 1 à 11, comme additifs dans les peintures, les revêtements, les encres d'impression, les revêtements protecteurs, les revêtements spéciaux dans les applications électroniques ou optiques, les résines photosensibles, les revêtements antireflets supérieurs ou les revêtements antireflets inférieurs, les solutions de révélateur et les solutions de rinçage et les résines photosensibles pour les procédés photo-lithographiques, les produits cosmétiques, les substances agrochimiques, les encaustiques, les revêtements photographiques ou les revêtements pour les éléments optiques.

13. Composition comprenant un composé selon l'une ou plusieurs parmi les revendications 1 à 11, et un véhicule qui est convenable pour l'application respective, et éventuellement d'autres substances actives spécifiques.

14. Composition selon la revendication 13, **caractérisée en ce que** la composition englobe les préparations de peintures et de revêtements, les compositions d'extinction d'incendies, les lubrifiants, les compositions de lavage et de nettoyage, les dégivreurs, les solutions de révélateur et les solutions de rinçage et les résines photosensibles pour les procédés photo-lithographiques, les produits cosmétiques, les substances agrochimiques, les encaustiques ou les compositions d'hydrophobie pour la finition des textiles ou le traitement du verre.

Abbildung 1: dynamische Oberflächenspannungen γ des Tensids aus Beispiel 1

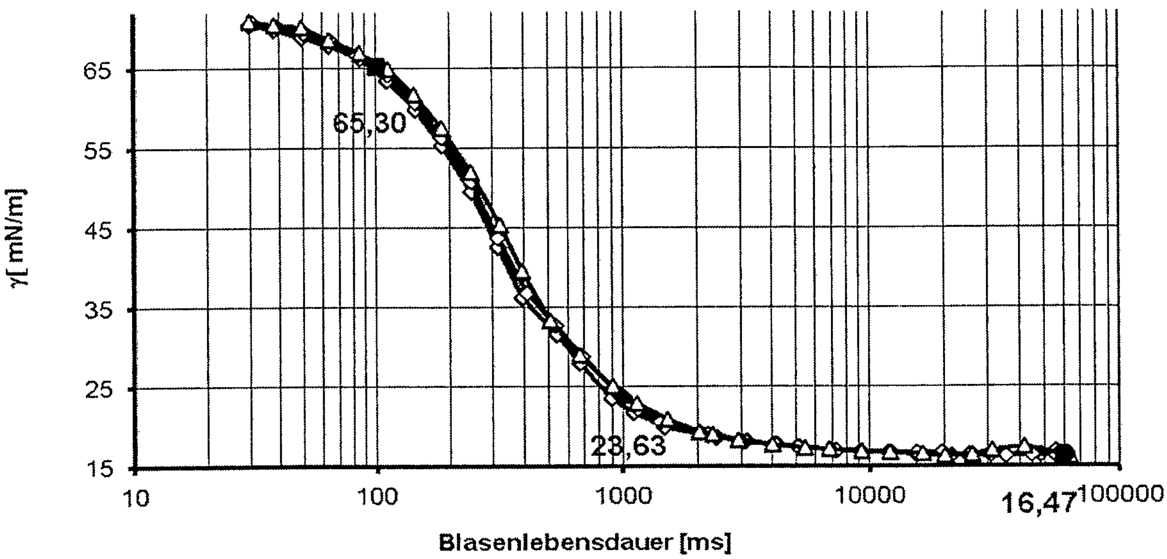

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03010128 A
- JP 2001133984 A
- JP 9111286 A
- WO 2006072401 A
- WO 2010003567 A
- US 7635789 B
- US 20080093582 A
- JP 2004018394 A
- WO 2010002623 A
- US 7737307 B
- EP 1522536 A
- WO 2010002622 A
- US 4968599 A
- US 4988610 A
- US 6890608 B
- WO 2009149807 A
- WO 2010149262 A
- WO 2011082770 A
- WO 2012084118 A
- WO 2014012661 A


### In der Beschreibung aufgeführte Nicht-Patentliteratur


- **G. L. KENNEDY, JR. ; J. L. BUTENHOFF ; G. W. OLSEN ; J. C. O'CONNOR ; A. M. SEACAT ; R. G. PERKINS ; L. B. BIEGEL ; S. R. MURPHY ; D. G. FARRAR.** *Critical Reviews in Toxicology,* 2004, vol. 34, 351-384
- **A.R. PITT et al.** *Colloids and Surfaces A: Physicochemical and Engineering Aspects,* 1996, vol. 114, 321-335
- **A.R. PITT.** *Progr. Colloid Polym. Sci,* 1997, vol. 103, 307-317
- **Z.-T. LIU et al.** *Ind. Eng. Chem. Res.,* 2007, vol. 46, 22-28
- **A. A. Il'IN et al.** *Russian Journal of Applied Chemistry,* 2007, vol. 80 (3), 405-418
- Atmospheric Fate of various fluorocarbons. **YI TANG.** Master Thesis. Massachusetts Institute of Technology, 1993